# EUROPEAN PATENT APPLICATION

(11) **EP 4 659 750 A1**
(43) Date of publication of application: **10.12.2025**
(21) Application number: 24749726.6
(22) Date of filing: 01.02.2024
(51) Int. Cl.: A61K 31/439, C07D 487/08, C07D 471/08, A61P 31/04

(54) **PHARMACEUTICAL COMPOSITION CONTAINING ß-LACTAMASE INHIBITOR AND USE THEREOF**

(30) Priority: 02.02.2023 CN 202310076617
(71) Applicant: Evopoint Biosciences Co., Ltd., Suzhou, Jiangsu 215000 (CN)
(72) Inventor: CHEN, Xi, Suzhou, Jiangsu 215000 (CN); LIU, Xiao, Suzhou, Jiangsu 215000 (CN); WU, Yuchuan, Suzhou, Jiangsu 215000 (CN); WENG, Qiuping, Suzhou, Jiangsu 215000 (CN); ZHU, Jinlian, Suzhou, Jiangsu 215000 (CN); XU, Yuanchang, Suzhou, Jiangsu 215000 (CN); SHEN, Yiwei, Suzhou, Jiangsu 215000 (CN); CHEN, Meng, Suzhou, Jiangsu 215000 (CN); ZHOU, Hongwei, Suzhou, Jiangsu 215000 (CN); MAO, Gecheng, Suzhou, Jiangsu 215000 (CN); WANG, Wengui, Suzhou, Jiangsu 215000 (CN)
(74) Representative: Engelhard, Markus
(86) International application number: PCT/CN2024/075190
(87) International publication number: WO 2024/160247

(57) **Abstract**

Disclosed in the present invention are a pharmaceutical composition containing a β-lactamase inhibitor and the use thereof, and specifically disclosed is a pharmaceutical composition A. The active ingredients of the pharmaceutical composition A comprise a substance A and a substance B, wherein the substance A is compound (1) or a solvate thereof, the substance B is imipenem or a solvate thereof, and the mass ratio of the substance B to the substance A is 1:1 to 10:1. The mass of the substance B is calculated on the basis of imipenem, and the mass of the substance A is calculated on the basis of the compound 1. The pharmaceutical composition of the present invention can effectively inhibit various bacteria that are resistant to β-lactam antibiotics, and can significantly increase the solubility of the compound 1 in an aqueous solution and improve the stability thereof.

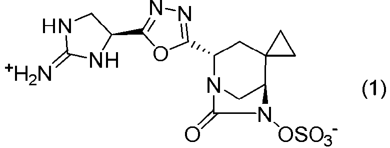

## Description

This application claims priority to Chinese Patent Application No. 2023100766170 filed on February 2, 2023, which is incorporated in this application by reference in its entirety.

### TECHNICAL FIELD

The present invention belongs to the field of pharmaceutical preparations, and in particular, relates to a pharmaceutical composition containing a β-lactamase inhibitor and use thereof.

### BACKGROUND ART

β-lactam antibiotics have always been the preferred choice of antibacterial agents in clinical practice. At present, however, the efficacy of related antibiotics is affected by bacterial β-lactamases, which can cause resistance to penicillins, extended-spectrum cephalosporins, monolactams and carbapenems.

β-lactamase inhibitors can delay or inhibit the degradation of β-lactam antibiotics and restore the sensitivity of bacteria, that produce resistance to β-lactam antibiotics, to the β-lactam antibiotics. At present, the β-lactamase inhibitors are used in combination with the β-lactam antibiotics in clinical practice to inactivate the hydrolysis activity of β-lactamase for the β-lactam antibiotics, so as to enhance the sensitivity of bacteria to the β-lactam antibiotics and thus reduce or overcome the problem of drug resistance. Currently, there are a variety of β-lactamase inhibitors on sale on the market, for example, clavulanic acid, tazobactam, avibactam, relebactam, etc. However, these β-lactamase inhibitors show a less satisfactory inhibitory effect against β-lactamase. Hence, there is an urgent need for a new β-lactamase inhibitor, which is anticipated to be combined with the β-lactam antibiotics to treat infections caused by bacteria resistant to the β-lactam antibiotics.

The World Health Organization (WHO) has listed carbapenem-resistant Enterobacteriaceae (CRE), carbapenem-resistant Pseudomonas aeruginosa (CRPA) and carbapenem-resistant Acinetobacter baumannii (CRAB) as drug-resistant bacteria with serious threats. It is urgent to solve this by developing a new generation of medicaments resistant to drug-resistance bacteria. Evopoint Biosciences has independently developed an innovative β-lactamase inhibitor, compound 1, specially targeting the drug resistance of bacteria to solve the problem that existing β-lactamase inhibitors are ineffective against CRE, CRPA and CRAB. The compound 1 can effectively inhibit OXA β-lactamases in vitro, which contribute to the main mechanism of Acinetobacter baumannii resistance to carbapenems, while similar products on the market or under development lack the inhibitory activity against OXA.

An international application WO2019144912A discloses a β-lactamase inhibitor or an ester, a stereoisomer and a pharmaceutically acceptable salt thereof and a preparation method therefor, as well as a pharmaceutical composition containing the β-lactamase inhibitor or the ester, stereoisomer and pharmaceutically acceptable salt therefor. A compound 1 involved therein is shown as below:

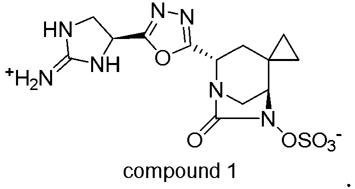

The compound 1 is a small-molecule insoluble drug with low solubility in water (0.6 mg/mL). Furthermore, it is susceptible to precipitation during storage and use, and has poor stability, which is less conducive to its combined use with other antibacterial drugs. So far, there is no pharmaceutical composition containing the compound 1 that can effectively inhibit a variety of bacteria resistant to β-lactam antibiotics. Moreover, there is an urgent need to develop an injection of the compound 1 to significantly increase its solubility and then stability in aqueous solution.

### SUMMARY OF THE INVENTION

The present invention aims to provide a pharmaceutical composition containing a β-lactamase inhibitor and use thereof, in order to solve the technical problem that there is no such a pharmaceutical composition containing a compound 1 that can effectively inhibit multiple bacteria in the art. The pharmaceutical composition of the present invention can effectively inhibit various bacteria that are resistant to β-lactam antibiotics, and can significantly increase the solubility and then stability of the compound 1 in an aqueous solution.

The present invention solves the above technical problem by means of technical solutions as follows.

The present invention provides a pharmaceutical composition A, wherein active ingredients of the pharmaceutical composition A comprise a substance A and a substance B;
the substance A is a compound 1 or a solvate thereof;
the substance B is imipenem or a solvate thereof;
a mass ratio of the substance B to the substance A is 1:1 to 10:1; the substance B has a mass calculated on the basis of the imipenem; and the substance A has a mass calculated on the basis of the compound 1.

In some embodiments, the active ingredients of the pharmaceutical composition A consist of the substance A and the substance B.

In some embodiments, the mass ratio of the substance B to the substance A is 1:1, 2:1, 3:1, 4:1, 5:1, 7:1 or 10:1.

In some embodiments, the mass ratio of the substance B to the substance A is 1:1 to 4:1.

In some embodiments, the mass ratio of the substance B to the substance A is 1:1 to 2:1.

In some embodiments, the mass ratio of the substance B to the substance A is 2:1.

In some embodiments, in the pharmaceutical composition A, the compound 1 has a mass percentage of 2% to 15%, preferably 5% to 10%, for example 6.44%, 6.50%, 6.97%, 7.29% or 8.93%.

In some embodiments, in the pharmaceutical composition A, the imipenem has a mass percentage of 5% to 20%, preferably 8% to 15%.

In some embodiments, in the pharmaceutical composition A, the substance B is imipenem monohydrate.

In some embodiments, in the pharmaceutical composition A, the substance B is imipenem monohydrate, and the imipenem monohydrate has a mass percentage of preferably 9% to 16%, for example, 13.81%, 13.68%, 15.45%, 13.67%, 14.80% or 9.48%.

In some embodiments, in the pharmaceutical composition A, the substance A is the compound 1.

In some embodiments, in the pharmaceutical composition A, the pharmaceutical composition A may further comprises a pharmaceutically acceptable excipient.

In some embodiments, in the pharmaceutical composition A, the pharmaceutically acceptable excipient comprises a solubilizer.

In some embodiments, in the pharmaceutical composition A, the pharmaceutically acceptable excipient further comprises one or two of a filler and a pH regulator.

In some embodiments, in the pharmaceutical composition A, the filler is selected from one or more of mannitol, lactose, sucrose, microcrystalline cellulose, glucose, fructose, povidone and trehalose, preferably mannitol and/or povidone.

In some embodiments, in the pharmaceutical composition A, the filler has a mass percentage of 0.3% to 70%, for example, 30% to 70% (preferably 35% to 50%), for example, 0.33%, 1.29% or 40.82%.

In some embodiments, in the pharmaceutical composition A, the solubilizer is selected from one or more of sodium sulphobutylether-β-cyclodextrin (SBECD), sodium hydroxypropyl-β-cyclodextrin (HP-β-CD), meglumine and polysorbate 80, preferably sodium sulphobutylether-β-cyclodextrin and/or sodium hydroxypropyl-β-cyclodextrin , more preferably sodium sulphobutylether-β-cyclodextrin.

In some embodiments, in the pharmaceutical composition A, the solubilizer has a mass percentage of 20% to 70%, for example, 20.41%, 64.38%, 62.73%, 65.03%, 64.40% or 71.40%, preferably 50% to 70%.

In some embodiments, in the pharmaceutical composition A, a mass ratio of the substance A to the solubilizer is 1:(2 to 13), for example 1:2.8, 1:5, 1:7.5, 1:8, 1:9, 1:10 or 1:12.5, preferably 1:(10 to 13), and the substance A has a mass calculated on the basis of the compound 1.

In some embodiments, in the pharmaceutical composition A, the pH regulator is selected from one or more of anhydrous citric acid, sodium hydroxide, sodium bicarbonate, anhydrous disodium hydrogen phosphate, sodium dihydrogen phosphate, disodium tartrate, maleic acid, magnesium hydroxide and calcium hydroxide, and preferably selected from one or more of sodium bicarbonate, sodium hydroxide and anhydrous citric acid.

In some embodiments, in the pharmaceutical composition A, the pH regulator has a mass percentage of 0% to 2.0%, for example, 0.52%, 0.58%, 0.70%, 0.71% or 1.83%, preferably, 0.3% to 0.7%.

In some embodiments, the pharmaceutical composition A exists as an injection (for example, lyophilized powder).

The present invention further provides use of the pharmaceutical composition A in preparation of a medicament for inhibiting bacteria, wherein the bacteria are resistant to β-lactam antibiotics.

In some embodiments, the bacteria are resistant to β-lactam antibiotics; and the bacteria resistant to β-lactam antibiotics are preferably selected from one or more of *Pseudomonas aeruginosa*, *Acinetobacter* baumannii and *Klebsiella pneumoniae.*

The present invention further provides use of the substance A in preparation of a medicament for inhibiting bacteria, wherein the substance A is used in combination with the substance B; a mass ratio of the substance B to the substance A is defined as above; and the bacteria are defined as above.

The present invention further provides use of the substance B in preparation of a medicament for inhibiting bacteria, wherein the substance B is used in combination with the substance A; a mass ratio of the substance B to the substance A is defined as above; and the bacteria are defined as above.

The present invention further provides a pharmaceutical composition B, wherein active ingredients of the pharmaceutical composition B comprise a substance A, a substance B and a substance C;
the substance A is a compound 1 or a solvate thereof;
the substance B is imipenem or a solvate thereof;
the substance C is cilastatin sodium or a solvate thereof;
a mass ratio of the substance C to the substance B to the substance A is (1 to 10):(1to10):1; the substance C has a mass calculated on the basis of cilastatin; the substance B has a mass calculated on the basis of the imipenem; and the substance A has a mass calculated on the basis of the compound 1.

In some embodiments, the active ingredients of the pharmaceutical composition B consist of the substance A, the substance B and the substance C.

In some embodiments, the mass ratio of the substance C to the substance B to the substance A is 1:1:1, 2:2:1, 3:3:1, 4:4:1, 5:5:1, 7:7:1 or 10:10:1.

In some embodiments, the mass ratio of the substance C to the substance B to the substance A is (1 to 4):(1 to 4):1.

In some embodiments, the mass ratio of the substance C to the substance B to the substance A is (1 to 2):(1 to 2):1.

In some embodiments, the mass ratio of the substance C to the substance B to the substance A is 2:2:1.

In some embodiments, in the pharmaceutical composition B, the compound 1 has a mass percentage of 2% to 15%, preferably 5% to 10%, for example 6.44%, 6.50%, 6.97%, 7.29% or 8.93%.

In some embodiments, in the pharmaceutical composition B, the imipenem has a mass percentage of 5% to 20%, preferably 8% to 15%.

In some embodiments, in the pharmaceutical composition B, the cilastatin sodium has a mass percentage of 5% to 20%, preferably 8% to 15%, and the cilastatin sodium has a mass calculated on the basis of cilastatin.

In some embodiments, in the pharmaceutical composition B, the substance B is imipenem monohydrate, and the imipenem monohydrate has a mass percentage of preferably 9% to 16%, for example, 13.81%, 13.68%, 15.45%, 13.67%, 14.80% or 9.48%.

In some embodiments, in the pharmaceutical composition B, the substance A is the compound 1.

In some embodiments, in the pharmaceutical composition B, the substance B is imipenem monohydrate.

In some embodiments, in the pharmaceutical composition B, the substance C is the cilastatin sodium.

In some embodiments, in the pharmaceutical composition B, the substance C exists as amorphous cilastatin sodium, and the cilastatin sodium has a mass percentage of preferably 9% to 16%, for example, 13.81%, 13.68%, 9.48%, 13.67%, 14.80% or 15.45%.

In some embodiments, in the pharmaceutical composition B, the pharmaceutical composition B may further comprises a pharmaceutically acceptable excipient.

In some embodiments, in the pharmaceutical composition B, the pharmaceutically acceptable excipient comprises a solubilizer.

In some embodiments, in the pharmaceutical composition B, the pharmaceutically acceptable excipient further comprises one or two of a filler and a pH regulator.

In some embodiments, in the pharmaceutical composition B, the filler is selected from one or more of mannitol, lactose, sucrose, microcrystalline cellulose, glucose, fructose, povidone and trehalose, preferably mannitol and/or povidone.

In some embodiments, in the pharmaceutical composition B, the filler has a mass percentage of 0.3% to 70%, for example, 30% to 70% (preferably 35% to 50%), for example, 0.33%, 1.29% or 40.82%.

In some embodiments, in the pharmaceutical composition B, the solubilizer is selected from one or more of sodium sulphobutylether-β-cyclodextrin (SBECD), sodium hydroxypropyl-β-cyclodextrin (HP-β-CD), meglumine and polysorbate 80, preferably sodium sulphobutylether-β-cyclodextrin and sodium hydroxypropyl-β-cyclodextrin , more preferably sodium sulphobutylether-β-cyclodextrin.

In some embodiments, in the pharmaceutical composition B, the solubilizer has a mass percentage of 20% to 70%, for example, 20.41%, 64.38%, 62.73%, 65.03%, 64.40% or 71.40%, preferably 50% to 70%.

In some embodiments, in the pharmaceutical composition B, a mass ratio of the substance A to the solubilizer is 1:(2 to 13), for example 1:2.8, 1:5, 1:7.5, 1:8, 1:9, 1:10 or 1:12.5, preferably 1:(10 to 13), and the substance A has a mass calculated on the basis of the compound 1.

In some embodiments, in the pharmaceutical composition B, the pH regulator is selected from one or more of anhydrous citric acid, sodium hydroxide, sodium bicarbonate, anhydrous disodium hydrogen phosphate, sodium dihydrogen phosphate, disodium tartrate, maleic acid, magnesium hydroxide and calcium hydroxide, and preferably selected from one or more of sodium bicarbonate, anhydrous citric acid and sodium hydroxide.

In some embodiments, in the pharmaceutical composition B, the pH regulator has a mass percentage of 0% to 2.0%, for example, 0.52%, 0.58%, 0.70%, 0.71% or 1.83%, preferably 0.3% to 0.7%.

In some embodiments, the pharmaceutical composition B comprises the compound 1, the sodium sulphobutylether-β-cyclodextrin, the mannitol, the imipenem monohydrate, the cilastatin sodium and the sodium bicarbonate.

In some embodiments, the pharmaceutical composition B comprises the compound 1, the sodium sulphobutylether-β-cyclodextrin, the anhydrous citric acid, the sodium hydroxide, the imipenem monohydrate, the cilastatin sodium and the sodium bicarbonate.

In some embodiments, the pharmaceutical composition B comprises the compound 1, the sodium sulphobutylether-β-cyclodextrin, the imipenem monohydrate, the cilastatin sodium and the sodium bicarbonate.

In some embodiments, the pharmaceutical composition B comprises the compound 1, the sodium sulphobutylether-β-cyclodextrin, the povidone, the imipenem monohydrate, the cilastatin sodium and the sodium bicarbonate.

In some embodiments, the pharmaceutical composition B comprises 7.29% of the compound 1, 20.41% of the sodium sulphobutylether-β-cyclodextrin, 40.82% of the mannitol, 15.45% of the imipenem monohydrate, 15.45% of the cilastatin sodium and 0.58% of the sodium bicarbonate, in percentage by mass.

In some embodiments, the pharmaceutical composition B comprises 6.44% of the compound 1, 64.38% of the sodium sulphobutylether-β-cyclodextrin, 0.99% of the anhydrous citric acid, 0.32% of the sodium hydroxide, 13.67% of the imipenem monohydrate, 13.67% of the cilastatin sodium and 0.52% of the sodium bicarbonate, in percentage by mass.

In some embodiments, the pharmaceutical composition B comprises 6.97% of the compound 1, 62.73% of the sodium sulphobutylether-β-cyclodextrin, 14.80% of the imipenem monohydrate, 14.80% of the cilastatin sodium and 0.70% of the sodium bicarbonate, in percentage by mass. In some embodiments, the pharmaceutical composition B comprises 8.93% of the compound 1, 71.40% of the sodium sulphobutylether-β-cyclodextrin, 9.48% of the imipenem monohydrate, 9.48% of the cilastatin sodium and 0.71% of the sodium bicarbonate, in percentage by mass.

In some embodiments, the pharmaceutical composition B comprises 6.50% of the Compound 1, 65.03% of the sodium sulphobutylether-β-cyclodextrin, 0.33% of the povidone, 0.52% of the sodium bicarbonate, 13.81% of the imipenem monohydrate, and 13.81% of the cilastatin sodium, in percentage by mass.

In some embodiments, the pharmaceutical composition B comprises 6.44% of the Compound 1, 64.40% of the sodium sulphobutylether-β-cyclodextrin, 1.29% of the povidone, 0.52% of the sodium bicarbonate, 13.68% of the imipenem monohydrate, and 13.68% of the cilastatin sodium, in percentage by mass.

In some embodiments, the pharmaceutical composition B exists as an injection.

In some embodiments, when the pharmaceutical composition B exists as an injection, the imipenem/cilastatin/compound 1 in the pharmaceutical composition B has a content of 250 mg/250 mg/125 mg or 500 mg/500 mg/250 mg.

The present invention further provides use of the pharmaceutical composition B in preparation of a medicament for inhibiting bacteria, wherein the bacteria are resistant to β-lactam antibiotics.

In some embodiments, the bacteria are resistant to β-lactam antibiotics; and the bacteria resistant to β-lactam antibiotics are preferably selected from one or more of *Pseudomonas aeruginosa, Acinetobacter* baumannii and *Klebsiella pneumoniae.*

The present invention further provides use of the substance A in preparation of a medicament for inhibiting bacteria, wherein the substance A is used in combination with the substance B and the substance C; a mass ratio of the substance C to the substance B to the substance A is defined as above; and the bacteria are defined as above.

The present invention further provides use of the substance B in preparation of a medicament for inhibiting bacteria, wherein the substance B is used in combination with the substance A and the substance C; a mass ratio of the substance C to the substance B to the substance A is defined as above; and the bacteria are defined as above.

The present invention further provides use of the substance C in preparation of a medicament for inhibiting bacteria, wherein the substance C is used in combination with the substance A and the substance B; a mass ratio of the substance C to the substance B to the substance A is defined as above; and the bacteria are defined as above.

In the present invention, the imipenem monohydrate has a structural formula shown below:

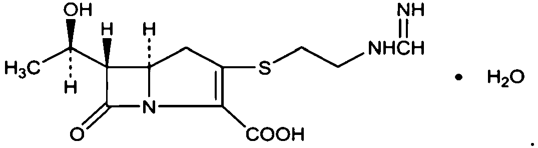

In the present invention, the cilastatin sodium has a structural formula shown below: .

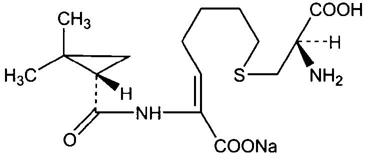

In the present invention, the compound 1 has a structural formula shown below:

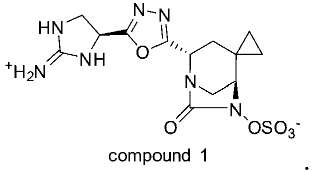

In the present invention, the term "solvate" refers to a substance formed by the combination of a compound and a solvent (including but not limited to water, methanol, ethanol, etc.). The solvates are classified into stoichiometric solvates and non-stoichiometric solvates. The solvates include but are not limited to monohydrates.

In accordance with the general knowledge in the art, the above-mentioned preferred conditions can be arbitrarily combined to arrive at the preferred examples of the present invention.

The reagents and raw materials used in the present invention are commercially available.

The present invention has the following positive effects: the pharmaceutical composition of the present invention can effectively inhibit various bacteria that are resistant to β-lactam antibiotics, and can significantly increase the solubility and then stability of the compound 1 in an aqueous solution.

### DETAILED DESCRIPTION OF THE PREFERRED EMBODIMENTS

The compound 1 used in the examples described below was provided by Bellen Chemistry Co. Ltd (China) with batch number 18090301.

The male mice used in the examples described below were provided by Charles River UK and were specific pathogen-free (SPF grade).

In the examples described below, culture load data were analyzed using StatsDirect software version 3.1.14 by means of an appropriate nonparametric statistical model (Kruskal-Wallis with Conover-Inman for all pairwise comparisons between groups).

The solubility test method for the compound 1 (or the content test method of the compound 1) in the Examples 6, 7, and 10 below is shown in the table below:

| | | | |
|---|---|---|---|
| Instrument | High-performance liquid chromatograph equipped with a UV detector | | |
| Chromatographic column | Waters Atlantis T3, 4.6 mm × 150 mm, 3 µm or other C18 chromatographic columns with equivalent column efficiency | | |
| Mobile phase | Mobile phase A: water-acetonitrile-methanol-0.05% phosphoric acid (990:5:5:0.5) | | |
| | Mobile phase B: acetonitrile-methanol-0.05% phosphoric acid (500:500:0.5) | | |
| Flow rate | 1.0 ml/min | Measurement wavelength | 210nm |
| Detector model | UV | Collection time | 30 minutes |
| Column temperature | 40°C | Injection volume | 10µl |

| | Time (minutes) | A (%) | B (%) |
|---|---|---|---|
| Operating gradient | 0 | 100 | 0 |
| | 10 | 90 | 10 |
| | 20 | 5 | 95 |
| | 25 | 5 | 95 |
| | 25.1 | 100 | 0 |
| | 30 | 100 | 0 |
| Probe rinse solution | Water-acetonitrile (90:10): mix 100 ml of acetonitrile and 900 ml of water homogenously, and sonicate the mixture for degassing to obtain the probe rinse solution. | | |
| Preparation of mobile phases | Mobile phase A: add 5 ml of acetonitrile and 5 ml of methanol to 990 ml of Milli-Q water, then add 0.5 ml of phosphoric acid, and mix them homogenously. | | |
| | Mobile phase B: add 500 ml of acetonitrile and 500 ml of methanol, then add 0.5 ml of phosphoric acid, and mix them homogenously. | | |
| Blank solution (diluent) | Ultra-pure water | | |
| Control solution | Precisely weigh 25 mg of control compound 1, place it in a 50 ml volumetric flask, add approximately 5 ml of acetonitrile, sonicate the mixture while shaking for about 5 minutes, then add approximately 30 ml of Milli-Q water, and sonicate the mixture for complete dissolution. | | |
| | Dilute to the mark with Milli-Q water and mix them homogenously. | | |
| | Prepare two parallel samples, labeled as STD#1 and STD#2. | | |
| Test solution | Take one bottle of sample and remove the seal cap. Inject 10 ml of water into the sample bottle by using a syringe, to dissolve the sample (do not open the stopper). Remove the rubber stopper with caution, take care to avoid solution loss, and transfer the solution to a 50 ml volumetric flask. | | |
| | Wash the vial three times with 5 ml of water each time (make sure that no residue sample is left on the rubber stopper). Transfer all solutions into the same 50 ml volumetric flask, dilute with water to the mark, and mix them homogenously. Transfer 2.0 ml of the sample stock solution to a 20 ml volumetric flask, and dilute to the mark with the diluent, and mix them homogenously. (Concentration: 0.5 mg/mL) | | |
| Content | The content of compound 1 (calculated on the basis of C₁₃H₁₇N₇O₆S) | | |
| measurement limit | should be 90.0% to 110.0% of the labeled content. | | |

The test methods for related substances (e.g., total impurities) and water in the Examples 7 and 10 described below are shown in the table below:

### Test method for related substances:

| | | | |
|---|---|---|---|
| Instrument | High-performance liquid chromatograph equipped with a UV detector | | |
| Chromatographic column | Octadecylsilane bonded silica gel as a filler for Waters Xselect HSS T₃ 4.6 mm × 250 mm, 3.5 µm chromatographic column or other chromatographic columns with equivalent efficacy | | |
| Mobile phase | Mobile phase A: 10 mmol/L diammonium hydrogen phosphate solution (pH adjusted to 6.80 ± 0.05 with phosphoric acid)-methanol (99.5:0.5). | | |
| | Mobile phase B: acetonitrile-methanol (800:200) | | |
| Flow rate | 1.0 ml/min | Measurement wavelength | 210 nm |
| Detector model | UV | Collection time | 70 min |
| Column temperature | 30°C | Injection volume | 10 µl |

| | Time (minutes) | A (%) | B (%) |
|---|---|---|---|
| Operating gradient | 0 | 100 | 0 |
| | 20 | 90 | 10 |
| | 30 | 75 | 25 |
| | 50 | 20 | 80 |
| | 55 | 20 | 80 |
| | 55.1 | 100 | 0 |
| | 70 | 100 | 0 |
| Probe rinse solution | Water-methanol (90:10): mix 100 ml of methanol and 900 ml of water homogenously, and sonicate the mixture for degassing to obtain the probe rinse solution. | | |
| Preparation of mobile phases | Mobile phase A: weigh 1.32 g of diammonium hydrogen phosphate, add dissolve it in 1000 ml of purified water, adjust the pH to 6.8 with phosphoric acid, take 995 ml of the resultant, add 5 ml of methanol, mix them homogenously, and sonicate the mixture for degassing to obtain the mobile phase A. | | |
| | Mobile phase B: mix 800 ml of acetonitrile and 200 ml of methanol, and sonicate the mixture to obtain the mobile phase B. | | |
| Blank solution (diluent) | Ultra-pure water | | |
| Blank excipient solution | Accurately weigh 100 mg of the lyophilized powder of sodium sulfobutylether-β-cyclodextrin, add it to a 20 ml volumetric flask, and dissolve and dilute it with an appropriate amount of diluent to the mark. | | |
| Control stock solution | Accurately weigh about 10.0 mg of control compound 1 into a 100 ml volumetric flask, add an appropriate amount of diluent, sonicate the mixture in a 60°C water bath for dissolution, dilute the mixture to the mark with purified water, and shake the mixture homogenously. | | |
| 0.2% control solution | Accurately transfer 0.2 ml of the control stock solution into a 20 ml volumetric flask, add the diluent to fix the volume to the mark, and shake the mixture homogenously to obtain the 0.2% control solution. Prepare in duplicate in parallel. | | |
| Sensitivity solution | Accurately add 2.5ml of the control solution into a 10 ml volumetric flask, dilute the control solution to the mark with the diluent, and shake it homogenously. | | |
| Test solution | Take one bottle of sample and remove the seal cap. Inject 10 ml of water into the sample bottle by using a syringe, to dissolve the sample (do not open the stopper). Remove the rubber stopper with caution, take care to avoid solution loss, and transfer the solution to a 50 ml volumetric flask. | | |
| | Wash the vial three times with 5 ml of water each time (make sure that no residue sample is left on the rubber stopper). Transfer all solutions into the same 50 ml volumetric flask, dilute with water to the mark, and mix them homogenously. Transfer 2.0 ml of the sample stock solution to a 20 ml volumetric flask, and dilute to the mark with the diluent, and mix them homogenously. (Concentration: 0.5 mg/mL) | | |

Total impurities refer to all impurities except for compound 1, and the percentage of the total impurities is calculated as: 1-(peak area of compound 1/sum of total peak areas).

### Water determination method:

The pharmaceutical composition of the present invention was tested in accordance with the water determination method (in Chinese Pharmacopoeia 2020 Edition, Part 4, General Rules 0832, Method 1-2).

In the following examples, the tests are conducted following the steps below.
(1) Immunization of mice: All mice are injected intraperitoneally with 150 mg/kg cyclophosphamide 4 days before inoculation and 100 mg/kg cyclophosphamide 1 day before inoculation to reduce their neutrophils by immunosuppression. The immunosuppressive regimen leads to neutropenia, which begins 24 hours after the first administration via injection and lasts throughout the study. The mice are inoculated approximately 24 hours after the second administration of the immunosuppressant.
(2) Bacterial culture: All bacterial strains used for inoculation are prepared from overnight broth cultures. In brief, each strain is allowed to grow in cation-adjusted MuellerHinton broth in a shaking incubator (300 RPM) at 37°C. The overnight cultures are washed twice with phosphate buffer solution (PBS) and adjusted to the optimal concentration in PBS. The concentration of the bacterial solution is confirmed by plating samples on drug-free CLED agar.
(3) The mice are inoculated by injecting 50 µL of bacterial solution into the lateral thigh muscles on both sides under temporary general anesthesia with isoflurane inhalation. After inoculation, all mice are given appropriate analgesia and observed until they are fully recovered from anesthesia.
(4) Imipenem and Compound 1 are administered starting from 1 hour after inoculation and then every 2 hours, and are administered sequentially, first with a subcutaneous injection of imipenem or imipenem-cilastatin solution, followed by an intravenous injection of the solution of Compound 1.

For intravenous (IV) administration, the solution of Compound 1 is prepared using a Captisol vehicle, which is 25 mM acetate buffer (prepared with sodium acetate trihydrate) containing 10% w/v Captisol. The pH is adjusted to 5.0 with 2N acetic acid solution. A desired volume of buffer is added to the weighed sample, the desired concentration of Compound 1 is prepared in the buffer, which is then ultrasonically heated in a water bath until a clear solution was obtained. The administrated volume of Compound 1 is 5 mL/kg.

For subcutaneous (SC) administration, an imipenem solution or imipenem-cilastatin solution is prepared in sterile PBS by using imipenem monohydrate or imipenem monohydrate and cilastatin sodium. The desired concentration of imipenem is prepared by adding PBS to the solid powder, then sonicating the mixture in a water bath until a clear solution is obtained, and the administrated volume is 10 mL/kg.

### Example 1

The mice were divided into 15 groups, with 5 mice in each group. The test was carried out following the above steps, the concentration of the bacterial solution of *Pseudomonas aeruginosa* ACC00680 was 1.83×10⁵ colony forming unit (CFU) per gram of thigh tissues, and the administration was conducted according to the scheme in the table below.

| Group | Ratio | Administrati on | Dose (mg/kg /potion ) | Total dose (mg/kg) | Route of administra tion | Time after inoculat ion (h) | End of study (hours after inocula tion) | Numbe r of mice |
|---|---|---|---|---|---|---|---|---|
| 1 | Not applic able (N/A) | Pretreatment (Pre-Tx) | N/A | N/A | N/A | N/A | 1 | 5 |
| 2 | N/A | Vehicle | 0 | 0 | IV | | 9 | 5 |
| 3 | N/A | Compound 1 | 25 | 100 | IV | 1,3,5,7 | 9 | 5 |
| 4 | N/A | Compound 1 | 18.75 | 75 | IV | 1,3,5,7 | 9 | 5 |
| 5 | N/A | Compound 1 | 12.5 | 50 | IV | 1,3,5,7 | 9 | 5 |
| 6 | N/A | Imipenem | 25 | 100 | SC | 1,3,5,7 | 9 | 5 |
| 7 | 1:1 | Imipenem and Compound 1 | 25/25 | 100/10 0 | SC and IV | 1,3,5,7 | 9 | 5 |
| 8 | 3:1 | Imipenem and Compound 1 | 25/7.5 | 100/30 | SC and IV | 1,3,5,7 | 9 | 5 |
| 9 | 10:1 | Imipenem and Compound 1 | 25/2.5 | 100/10 | SC and IV | 1,3,5,7 | 9 | 5 |
| 10 | 1:1 | Imipenem and Compound 1 | 18.75/1 8.75 | 75/75 | SC and IV | 1,3,5,7 | 9 | 5 |
| 11 | 3:1 | Imipenem and Compound 1 | 18.75/6 .25 | 75/25 | SC and IV | 1,3,5,7 | 9 | 5 |
| 12 | 10:1 | Imipenem and Compound 1 | 18.75/1. 875 | 75/7.5 | SC and IV | 1,3,5,7 | 9 | 5 |
| 13 | 1:1 | Imipenem and Compound 1 | 12.5/12. 5 | 50/50 | SC and IV | 1,3,5,7 | 9 | 5 |
| 14 | 3:1 | Imipenem and Compound 1 | 12.5/4.1 6 | 50/16.6 7 | SC and IV | 1,3,5,7 | 9 | 5 |
| 15 | 10:1 | Imipenem and Compound 1 | 12.5/1.2 5 | 50/5 | SC and IV | 1,3,5,7 | 9 | 5 |

For Groups 2 to 15 above, animals were euthanized 9 hours after inoculation using an overdose of sodium pentobarbital, followed by confirmation of death by cervical dislocation. The thighs were dissected and weighed, then homogenized in an ice-cold sterile phosphate buffer using a Precellys microsphere homogenizer; and the homogenate was quantitatively cultured on drug-free agar (CLED or PSA) and incubated for 18-24 h at 37°C, followed by colony counting.

For the uninoculated Group 1 (control group) above, animals were euthanized with an overdose of sodium pentobarbital 1 hour after inoculation. As described above, bilateral thighs were dissected and quantitatively cultured for use as final samples.

The changes in log₁₀ CFU/g load for all administrated groups and the statistical significance versus Pre-Tx were summarized in the table below.

| Administration | Ratio | Group geometric mean (CFU/g) | Log₁₀ group geometric mean (CFU/g) | Log₁₀ standard deviation (CFU/g) | Change in Pre-Tx Log₁₀ load | P vs. Pre-Tx |
|---|---|---|---|---|---|---|
| Pre-Tx (1h) | N/A | 2.02×10⁵ | 5.30 | 0.55 | N/A | N/A |
| Vehicle | N/A | 1.49×10⁶ | 6.17 | 0.56 | 0.87 | < 0.0001 |
| Imipenem (100 mg/kg) | N/A | 1.08×10⁶ | 6.03 | 0.37 | 0.73 | < 0.005 |
| Compound 1 (100 mg/kg) | N/A | 4.52×10⁶ | 6.65 | 0.67 | 1.35 | < 0.0001 |
| Compound 1 (75 mg/kg) | N/A | 1.93×10⁶ | 6.29 | 0.71 | 0.98 | < 0.0001 |
| Compound 1 (50 mg/kg) | N/A | 4.63×10⁵ | 5.67 | 0.46 | 0.36 | Not Significant (NS) |
| Imipenem (100 mg/kg) + Compound 1 (100 mg/kg) | 1:1 | 1.94 | 0.29 | 0.91 | -5.02 | < 0.0001 |
| Imipenem (100 mg/kg) + Compound 1 (30 mg/kg) | 3:1 | 4.13×10² | 2.62 | 0.30 | -2.69 | < 0.0001 |
| Imipenem (100 mg/kg) + Compound 1 (10 mg/kg) | 10:1 | 1.50×10⁴ | 4.18 | 0.22 | -1.13 | < 0.005 |
| Imipenem (75 mg/kg) + Compound 1 (75 mg/kg) | 1:1 | 2.63×10² | 2.42 | 0.89 | -2.88 | < 0.0001 |
| Imipenem (75 mg/kg) + Compound 1 (25 mg/kg) | 3:1 | 8.94×10² | 2.95 | 0.36 | -2.35 | < 0.0001 |
| Imipenem (75 mg/kg) + Compound 1 (7.5 mg/kg) | 10:1 | 8.57× 103 | 3.93 | 0.52 | -1.37 | < 0.0001 |
| Imipenem (50 mg/kg) + Compound 1 (50 mg/kg) | 1:1 | 5.23×10² | 2.72 | 0.21 | -2.59 | < 0.0001 |
| Imipenem (50 mg/kg) + Compound 1 (16.7 mg/kg) | 3:1 | 2.63×10² | 2.42 | 0.43 | -2.88 | < 0.0001 |
| Imipenem (50 mg/kg) + Compound 1 (5 mg/kg) | 10:1 | 6.21×10³ | 3.79 | 0.69 | -1.51 | < 0.0001 |

When the total dose of imipenem was 100 mg/kg, 75 mg/kg and 50 mg/kg, the imipenem and compound 1 were co-administered at a dose ratio of 1:1, 3:1 or 10:1, which significantly reduced the thigh load to below a stagnant level.

In addition, compared with the groups administered with imipenem and compound 1 alone,the co-administration of imipenem and compound 1 at all dose ratios also significantly reduced the thigh load.

### Example 2

The mice were divided into 16 groups, with 5 mice in each group. The test was carried out following the above steps, the concentration of the bacterial solution of *Pseudomonas aeruginosa* ACC00680 was 1.83×10⁵ colony forming unit (CFU) per gram of thigh tissues, and the administration was conducted according to the scheme in the table below.

| Gro up | Rat io | Administra tion | Dose (mg/kg/po tion) | Total dose (mg/kg ) | Route of administra tion | Time after inoculat ion (h) | End of study (hours after inoculati on) | Num ber of mice |
|---|---|---|---|---|---|---|---|---|
| 1 | N/ A | Pre-Tx | N/A | N/A | N/A | N/A | 1 | 5 |
| 2 | N/ A | Vehicle | 0 | 0 | SC | 1, 3, 5, 7 | 9 | 5 |
| 3 | N/ A | Imipenem + Cilastatin | 24 + 24 | 96 + +96 | SC | 1, 3, 5, 7 | 9 | 5 |
| 4 | N/ A | Compound 1 | 24 | 96 | IV | 1, 3, 5, 7 | 9 | 5 |
| 5 | 1:1 | Imipenem + Cilastatin + Compound 1 | 24+24+24 | 96+96 +96 | SC and IV | 1, 3, 5, 7 | 9 | 5 |
| 6 | 2:1 | Imipenem + Cilastatin + Compound 1 | 24+24+12 | 96+96 +48 | SC and IV | 1, 3, 5, 7 | 9 | 5 |
| 7 | 4:1 | Imipenem + Cilastatin + Compound 1 | 24+24+6 | 96+96 +24 | SC and IV | 1, 3, 5, 7 | 9 | 5 |
| 8 | 1:1 | Imipenem + Cilastatin + Compound 1 | 16+16+16 | 64+64 +64 | SC and IV | 1, 3, 5, 7 | 9 | 5 |
| 9 | 2:1 | Imipenem + Cilastatin + Compound 1 | 16+16+8 | 64+64 +32 | SC and IV | 1, 3, 5, 7 | 9 | 5 |
| 10 | 4:1 | Imipenem + Cilastatin + Compound 1 | 16+16+4 | 64+64 +16 | SC and IV | 1, 3, 5, 7 | 9 | 5 |
| 11 | 1:1 | Imipenem + Cilastatin + Compound 1 | 12+12+12 | 48+48 +48 | SC and IV | 1, 3, 5, 7 | 9 | 5 |
| 12 | 2:1 | Imipenem + Cilastatin + Compound 1 | 12+12+6 | 48+48 +24 | SC and IV | 1, 3, 5, 7 | 9 | 5 |
| 13 | 4:1 | Imipenem + Cilastatin + Compound 1 | 12+12+3 | 48+48 +12 | SC and IV | 1, 3, 5, 7 | 9 | 5 |
| 14 | 1:1 | Imipenem + Cilastatin + Compound 1 | 8+8+8 | 32+32+ 32 | SC and IV | 1, 3, 5, 7 | 9 | 5 |
| 15 | 2:1 | Imipenem + Cilastatin + Compound 1 | 8+8+4 | 32+32+ 16 | SC and IV | 1, 3, 5, 7 | 9 | 5 |
| 16 | 4:1 | Imipenem + Cilastatin + Compound 1 | 8+8+2 | 32+32+ 8 | SC and IV | 1, 3, 5, 7 | 9 | 5 |

For Groups 2 to 16 above, animals were euthanized 9 hours after inoculation using an overdose of sodium pentobarbital, followed by confirmation of death by cervical dislocation. The thighs were dissected and weighed, then homogenized in an ice-cold sterile phosphate buffer using a Precellys microsphere homogenizer; and the homogenate was quantitatively cultured on drug-free agar (CLED or PSA) and incubated for 18-24 h at 37°C, followed by colony counting.

For the uninoculated Group 1 (control group) above, animals were euthanized with an overdose of sodium pentobarbital 1 hour after inoculation. As described above, bilateral thighs were dissected and quantitatively cultured for use as final samples.

The changes in log₁₀ CFU/g load for all administrated groups and the statistical significance versus Pre-Tx were summarized in the table below.

| Administration | Ratio | Group geometric mean (CFU/g) | Log₁₀ group geometric mean (CFU/g) | Log₁₀ standard deviation (CFU/g) | Change in Pre-Tx Log₁₀ loading (CFU/g) | P vs. Pre-Tx |
|---|---|---|---|---|---|---|
| Pre-Tx (1h) | N/A | 1.15×10⁵ | 5.06 | 0.28 | N/A | N/A |
| Vehicle | N/A | 4.14×10⁶ | 5.62 | 0.52 | 0.56 | NS |
| Imipenem (96 mg/kg) + Cilastatin (96 mg/kg) | N/A | 5.25×10⁵ | 5.72 | 0.23 | 0.66 | NS |
| Compound 1 (96 mg/kg) | N/A | 3.21×10⁷ | 7.51 | 0.37 | 2.45 | < 0.005 |
| Imipenem (96 mg/kg) + Cilastatin (96 mg/kg) + Compound 1 (96 mg/kg) | 1:1 | 2.72×10² | 2.43 | 0.88 | 2.63 | < 0.0001 |
| Imipenem (96 mg/kg) + Cilastatin (96 mg/kg) + Compound 1 (48 mg/kg) | 2:1 | 1.56×10² | 2.19 | 1.19 | 2.87 | < 0.0001 |
| Imipenem (96 mg/kg) + Cilastatin (96 mg/kg) + Compound 1 (24 mg/kg) | 4:1 | 1.10×10² | 2.04 | 1.11 | -3.02 | < 0.0001 |
| Imipenem (64 mg/kg) + Cilastatin (64 mg/kg) + Compound 1 (64 mg/kg) | 1:1 | 3.96×10¹ | 1.60 | 1.43 | -3.46 | < 0.0001 |
| Imipenem (64 mg/kg) + Cilastatin (64 mg/kg) + Compound 1 (32 mg/kg) | 2:1 | 4.12×10³ | 3.61 | 0.14 | -1.45 | < 0.005 |
| Imipenem (64 mg/kg) + Cilastatin (64 mg/kg) + Compound 1 (16 mg/kg) | 4:1 | 4.00×10³ | 3.60 | 0.19 | -1.46 | < 0.005 |
| Imipenem (48 mg/kg) + Cilastatin (48 mg/kg) + Compound 1 (48 mg/kg) | 1:1 | 1.15×10³ | 3.06 | 1.09 | -2.00 | < 0.0001 |
| Imipenem (48 mg/kg) + Cilastatin (48 mg/kg) + Compound 1 (24 mg/kg) | 2:1 | 2.12×10³ | 3.33 | 0.37 | -1.73 | < 0.005 |
| Imipenem (48 mg/kg) + Cilastatin (48 mg/kg) + Compound 1 (12 mg/kg) | 4:1 | 1.32×10³ | 3.12 | 0.45 | -1.94 | < 0.005 |
| Imipenem (32 mg/kg) + Cilastatin (32 mg/kg) + Compound 1 (32 mg/kg) | 1:1 | 1.93×10³ | 3.29 | 0.38 | -1.77 | < 0.0001 |
| Imipenem (32 mg/kg) + Cilastatin (32 mg/kg) + Compound 1 (16 mg/kg) | 2:1 | 1.66×10³ | 3.22 | 0.67 | -1.84 | < 0.0001 |
| Imipenem (32 mg/kg) + Cilastatin (32 mg/kg) + Compound 1 (8 mg/kg) | 4:1 | 1.40×10² | 2.15 | 0.84 | -2.91 | < 0.0001 |

When the total dose of imipenem was 96 mg/kg, 48 mg/kg and 32 mg/kg, the imipenem and compound 1 were co-administered at a dose ratio of 1:1, 2:1 or 4:1, which significantly reduced the thigh load to below a stagnant level. At each total dose level, there was no significant correlation trend between the load reduction level and the dose ratio.

### Example 3

The mice were divided into 16 groups, with 5 mice in each group. The test was carried out following the above experimental steps, the inoculation amount of *Acinetobacter baumannii* AR0274 was 1.8×10⁶ CFU per gram of thigh tissue, and the administration was conducted according to the scheme in the table below.

| Group | Rati o | Administrat ion | Dose (mg/kg/ potion) | Total dose (mg/kg ) | Route of administrat ion | Time after inoculati on (h) | End of study (hours after inoculation ) | Nu mbe r of mice |
|---|---|---|---|---|---|---|---|---|
| 1 | N/A | Pre-Tx | N/A | N/A | N/A | N/A | 1 | 5 |
| 2 | N/A | Vehicle | 0 | 0 | SC | 1,3,5,7 | 9 | 5 |
| 3 | N/A | Imipenem | 24 | 96 | SC | 1,3,5,7 | 9 | 5 |
| 4 | N/A | Compound 1 | 24 | 96 | IV | 1,3,5,7 | 9 | 5 |
| 5 | 1:1 | Imipenem + Compound 1 | 24+24 | 96+96 | SC and IV | 1,3,5,7 | 9 | 5 |
| 6 | 2:1 | Imipenem + Compound 1 | 24+12 | 96+48 | SC and IV | 1,3,5,7 | 9 | 5 |
| 7 | 4:1 | Imipenem + Compound 1 | 24+6 | 96+24 | SC and IV | 1,3,5,7 | 9 | 5 |
| 8 | 1:1 | Imipenem + Compound 1 | 16+16 | 64+64 | SC and IV | 1,3,5,7 | 9 | 5 |
| 9 | 2:1 | Imipenem + Compound 1 | 16+8 | 64+32 | SC and IV | 1,3,5,7 | 9 | 5 |
| 10 | 4:1 | Imipenem + Compound 1 | 16+4 | 64+16 | SC and IV | 1,3,5,7 | 9 | 5 |
| 11 | 1:1 | Imipenem + Compound 1 | 8+8 | 32+32 | SC and IV | 1,3,5,7 | 9 | 5 |
| 12 | 2:1 | Imipenem + Compound 1 | 8+4 | 32+16 | SC and IV | 1,3,5,7 | 9 | 5 |
| 13 | 4:1 | Imipenem + Compound 1 | 8+2 | 32+8 | SC and IV | 1,3,5,7 | 9 | 5 |
| 14 | 1:1 | Imipenem + Compound 1 | 4+4 | 16+16 | SC and IV | 1,3,5,7 | 9 | 5 |
| 15 | 2:1 | Imipenem + Compound 1 | 4+2 | 16+8 | SC and IV | 1,3,5,7 | 9 | 5 |
| 16 | 4:1 | Imipenem + Compound 1 | 4+1 | 16+4 | SC and IV | 1,3,5,7 | 9 | 5 |

For Groups 2 to 16 above, animals were euthanized 9 hours after inoculation using an overdose of sodium pentobarbital, followed by confirmation of death by cervical dislocation. The thighs were dissected and weighed, then homogenized in an ice-cold sterile phosphate buffer using a Precellys microsphere homogenizer; and the homogenate was quantitatively cultured on drug-free agar (CLED or PSA) and incubated for 18-24 h at 37°C, followed by colony counting.

For the uninoculated Group 1 (control group) above, animals were euthanized with an overdose of sodium pentobarbital 1 hour after inoculation. As described above, bilateral thighs were dissected and quantitatively cultured for use as final samples.

The changes in log₁₀ CFU/g load for all administrated groups and the statistical significance versus Pre-Tx were summarized in the table below.

| Administration | Ratio | Group geometric mean (CFU/g) | Log₁₀ group geometric mean (CFU/g) | Log₁₀ standard deviation (CFU/g) | Change in Pre-Tx Log₁₀ loading (CFU/g) | P vs. Pre-Tx |
|---|---|---|---|---|---|---|
| Pre-Tx (1h) | N/A | 5.58×10⁵ | 5.75 | 0.44 | N/A | N/A |
| Vehicle | *N*/*A* | 9.64×10⁷ | 7.98 | 0.26 | 2.24 | < 0.0001 |
| Imipenem (96 mg/kg) | N/A | 3.91×10⁷ | 7.59 | 1.20 | 1.85 | < 0.0001 |
| Compound 1 (96 mg/kg) | N/A | 1.89×10⁷ | 7.28 | 0.84 | 1.53 | < 0.0001 |
| Imipenem (96 mg/kg) + Compound 1 (96 mg/kg) | 1:1 | 1.79×10⁴ | 4.25 | 0.29 | -1.49 | < 0.0001 |
| Imipenem (96 mg/kg) + Compound 1 (48 mg/kg) | 2:1 | 1.47×10⁴ | 4.17 | 0.46 | -1.58 | < 0.0001 |
| Imipenem (96 mg/kg) + Compound 1 (24 mg/kg) | 4:1 | 2.46×10⁴ | 4.39 | 0.34 | -1.35 | < 0.0001 |
| Imipenem (64 mg/kg) + Compound 1 (64 mg/kg) | 1:1 | 2.06×10⁴ | 4.31 | 0.29 | -1.43 | < 0.0001 |
| Imipenem (64 mg/kg) + Compound 1 (32 mg/kg) | 2:1 | 6.14x10⁴ | 4.79 | 0.70 | -0.96 | < 0.05 |
| Imipenem (64 mg/kg) + Compound 1 (16 mg/kg) | 4:1 | 4.21×10⁵ | 5.62 | 1.00 | -0.12 | NS |
| Imipenem (32 mg/kg) + Compound 1 (32 mg/kg) | 1:1 | 9.46×10⁵ | 5.98 | 0.89 | 0.23 | NS |
| Imipenem (32 mg/kg) + Compound 1 (16 mg/kg) | 2:1 | 5.16×10⁶ | 6.71 | 0.90 | 0.97 | NS |
| Imipenem (32 mg/kg) + Compound 1 (8 mg/kg) | 4:1 | 2.78×10⁷ | 7.44 | 0.26 | 1.70 | NS |
| Imipenem (16 mg/kg) + Compound 1 (16 mg/kg) | 1:1 | 3.15×10⁷ | 7.50 | 0.46 | 1.75 | NS |
| Imipenem (16 mg/kg) + Compound 1 (8 mg/kg) | 2:1 | 4.12×10⁷ | 7.61 | 0.21 | 1.87 | NS |
| Imipenem (16 mg/kg) + Compound 1 (4 mg/kg) | 4:1 | 6.36×10⁷ | 7.80 | 0.21 | 2.06 | NS |

When the total dose level of imipenem was 96 mg/kg, the imipenem and the Compound 1 were co-administered at a dose ratio of 1:1, 2:1, or 4:1, which significantly reduced the thigh load to below a stagnant level. A lower total dose of imipenem (64 mg/kg) also reduced the thigh load to below the stagnant level, but only at the combined dose ratios of 1:1 and 2:1. The total dose of imipenem at 32 mg/kg or 16 mg/kg did not show a reduction in load compared to the stagnant level.

In addition, compared with the groups administrated with the vehicle, imipenem or Compound 1, the combined administration of imipenem and Compound 1 at all dose ratios also significantly reduced the thigh load when the total doses of imipenem were 96 mg/kg and 64 mg/kg, but there was no significant reduction in thigh load when the imipenem was at a lower total dose level.

### Example 4

The mice were divided into 16 groups, with 5 mice in each group. The test was carried out following the above steps, the concentration of the bacterial solution of *Klebsiella pneumoniae* ARo113 was 4.0x10⁶ CFU per gram of thigh tissues, and the administration was conducted according to the scheme in the table below.

| Group | Ratio | Administration | Dose (mg/kg/ potion) | Total dose (mg/kg) | Route of administr ation | Time after inoculation (h) | End of study (hours after inoculation) | Nu mber of mice |
|---|---|---|---|---|---|---|---|---|
| 1 | N/A | Pre-Tx | N/A | N/A | N/A | N/A | 1 | 5 |
| 2 | N/A | Vehicle | 0 | 0 | IV | 1,3,5,7 | 9 | 5 |
| 3 | N/A | Imipenem | 24 | 96 | SC | 1,3,5,7 | 9 | 5 |
| 4 | N/A | Compound 1 | 24 | 96 | SC and IV | 1,3,5,7 | 9 | 5 |
| 5 | 1:1 | Imipenem + Compound 1 | 24+24 | 96+96 | SC and IV | 1,3,5,7 | 9 | 5 |
| 6 | 2:1 | Imipenem + Compound 1 | 24+12 | 96+48 | SC and IV | 1,3,5,7 | 9 | 5 |
| 7 | 4:1 | Imipenem + Compound 1 | 24+6 | 96+24 | SC and IV | 1,3,5,7 | 9 | 5 |
| 8 | 1:1 | Imipenem + Compound 1 | 8+8 | 32+32 | SC and IV | 1,3,5,7 | 9 | 5 |
| 9 | 2:1 | Imipenem + Compound 1 | 8+4 | 32+16 | SC and IV | 1,3,5,7 | 9 | 5 |
| 10 | 4:1 | Imipenem + Compound 1 | 8+2 | 32+8 | SC and IV | 1,3,5,7 | 9 | 5 |
| 11 | 1:1 | Imipenem + Compound 1 | 3+3 | 12+12 | SC and IV | 1,3,5,7 | 9 | 5 |
| 12 | 2:1 | Imipenem + Compound 1 | 3+1.5 | 12+6 | SC and IV | 1,3,5,7 | 9 | 5 |
| 13 | 4:1 | Imipenem + Compound 1 | 3+0.75 | 12+3 | SC and IV | 1,3,5,7 | 9 | 5 |
| 14 | 1:1 | Imipenem + Compound 1 | 1.5+1.5 | 6+6 | SC and IV | 1,3,5,7 | 9 | 5 |
| 15 | 2:1 | Imipenem + Compound 1 | 1.5+0.75 | 6+3 | SC and IV | 1,3,5,7 | 9 | 5 |
| 16 | 4:1 | Imipenem + Compound 1 | 1.5+0.375 | 6+1.5 | SC and IV | 1,3,5,7 | 9 | 5 |

For Groups 2 to 16 above, animals were euthanized 9 hours after inoculation using an overdose of sodium pentobarbital, followed by confirmation of death by cervical dislocation. The thighs were dissected and weighed, then homogenized in an ice-cold sterile phosphate buffer using a Precellys microsphere homogenizer; and the homogenate was quantitatively cultured on drug-free agar (CLED or PSA) and incubated for 12-16 h at 30°C, followed by colony counting.

For the uninoculated Group 1 (control group) above, animals were euthanized with an overdose of sodium pentobarbital 1 hour after inoculation. As described above, bilateral thighs were dissected and quantitatively cultured for use as final samples.

The changes in log₁₀ CFU/g load for all administrated groups and the statistical significance versus Pre-Tx were summarized in the table below.

| Administration | Ratio | Group geometric mean (CFU/g) | Log₁₀ group geometric mean (CFU/g) | Log₁₀ standard deviation (CFU/g) | Change in Pre-Tx Log₁₀ loading (CFU/g) | P vs. Pre-Tx |
|---|---|---|---|---|---|---|
| Pre-Tx (1h) | N/A | 1.58×10⁶ | 6.20 | 0.46 | N/A | N/A |
| Vehicle | N/A | 3.55×10⁷ | 7.55 | 0.56 | 1.35 | < 0.0001 |
| Imipenem (96 mg/kg) | N/A | 1.34×10⁷ | 7.13 | 0.51 | 0.93 | < 0.0001 |
| Compound 1 (96 mg/kg) | N/A | 2.06×10⁷ | 7.31 | 0.81 | 1.11 | < 0.0001 |
| Imipenem (96 mg/kg) + Compound 1 (96 mg/kg) | 1:1 | 4.38×10⁵ | 5.64 | 0.75 | -0.56 | < 0.0001 |
| Imipenem (96 mg/kg) + Compound 1 (48 mg/kg) | 2:1 | 5.87×10⁵ | 5.77 | 0.51 | -0.43 | < 0.0001 |
| Imipenem (96 mg/kg) + Compound 1 (24 mg/kg) | 4:1 | 3.45×10⁵ | 5.54 | 1.04 | -0.66 | < 0.0001 |
| Imipenem (32 mg/kg) + Compound 1 (32 mg/kg) | 1:1 | 1.31×10⁶ | 6.12 | 0.46 | -0.08 | < 0.0001 |
| Imipenem (32 mg/kg) + Compound 1 (16 mg/kg) | 2:1 | 5.96×10⁶ | 6.78 | 0.34 | 0.58 | < 0.05 |
| Imipenem (32 mg/kg) + Compound 1 (8 mg/kg) | 4:1 | 9.95×10⁶ | 7.00 | 0.37 | 0.80 | NS |
| Imipenem (12 mg/kg) + Compound 1 (12 mg/kg) | 1:1 | 2.92×10⁷ | 7.47 | 0.34 | 1.27 | NS |
| Imipenem (12 mg/kg) + Compound 1 (6 mg/kg) | 2:1 | 3.99×10⁷ | 7.60 | 0.20 | 1.40 | NS |
| Imipenem (12 mg/kg) + Compound 1 (3 mg/kg) | 4:1 | 2.16×10⁷ | 7.34 | 0.46 | 1.14 | NS |
| Imipenem (6 mg/kg) + Compound 1 (6 mg/kg) | 1:1 | 3.28×10⁷ | 7.52 | 0.45 | 1.32 | NS |
| Imipenem (6 mg/kg) + Compound 1 (3 mg/kg) | 2:1 | 1.58×10⁸ | 8.20 | 0.30 | 2.00 | NS |
| Imipenem (6 mg/kg) + Compound 1 (1.5 mg/kg) | 4:1 | 1.08×10⁸ | 8.03 | 0.39 | 1.83 | NS |

When the total dose level of imipenem was 96 mg/kg, the imipenem and the Compound 1 were co-administered at a dose ratio of 1:1, 2:1, or 4:1, in which case the thigh load was slightly reduced but significantly reduced to below a stagnant level. In the group administrated with low-dose imipenem (32 mg/kg, 12 mg/kg or 6 mg/kg), the thigh load was not observed to decrease below the stagnant level.

In addition, compared with the groups administrated with the vehicle, imipenem or Compound 1, the combined administration of imipenem and Compound 1 at all dose ratios also significantly reduced the thigh load when the total dose levels of imipenem were 96 mg/kg and 64 mg/kg, but there was no reduction in thigh load when the imipenem was at a lower total dose level.

### Example 5

A total of 15 bacterial strains (4 strains of *Pseudomonas aeruginosa*, 4 strains of *Klebsiella pneumoniae* and 7 strains of *Acinetobacter* baumannii, see the table below) were selected for dose study of Compound 1 in the mice neutropenic thigh infection model. In terms of administrated dose, imipenem was administered with a human-simulated dosing regimen (500 mg, q6h, infused for 60 minutes),and also combined with different doses of Compound 1.

| Isolated strain | Encoded β-lactamase |
|---|---|
| PSA 1844 | KPC-2, PDC-42 |
| PSA 1862 | GES-19, GES-20 |
| PSA 1866 | GES-20 |
| PSA 1869 | GES-19, GES-26 |
| ACB 160 | OXA-24, OXA-65 , TEM-1B |
| ACB 179 | ADC-25, OXA-23, OXA-223 |
| ACB 193 | OXA-23, OXA-82 |
| ACB 194 | ADC-25, OXA-23, OXA-82 |
| ACB 209 | OXA-65, OXA-24 |
| ACB 246 | ADC-33, OXA-23, OXA-82 |
| ACB 258 | ADC-222, OXA-23, OXA-95 |
| KP 648 | KPC-3 |
| KP 651 | KPC-2 |
| KP 741 | SHV-11, CTX-M-55, OXA-48 |
| KP 827 | OXA-48, CTX-M-15, OXA-1, SHV-11, TEM-1B |

### (1) Pseudomonas aeruginosa

At 0 h, the mean (± SD) bacterial load in the thighs of mice was 5.64 ± 0.41 log₁₀ CFU/thigh. Four isolated strains tested were all sufficiently proliferated in the neutropenic thigh infection model; and in the control mice administrated with an untreated vehicle, the mean increase in bacterial load at 24 h was 3.33 ± 0.62 log₁₀ CFU/thigh. In vitro resistance to imipenem was confirmed in the in vivo study of these four strains; and in the monotherapy group with the human-simulated imipenem dosing regimen, the mean increase in bacterial load was 2.64 ± 1.18 log₁₀ CFU/thigh. With the human-simulated dosing regime with the imipenem/Compound 1 of 500/250 mg (2:1), the four isolated strains of *Pseudomonas aeruginosa* were all killed at 2-log.

### (2) Acinetobacter baumannii

At 0 h, the mean bacterial load in the thighs of mice was 5.97 ± 0.18 log₁₀ CFU/thigh. Seven isolated strains tested were all sufficiently proliferated in the neutropenic thigh infection model; and in the control mice administrated with an untreated vehicle, the mean increase in bacterial load at 24 h was 2.32 ± 0.84 log₁₀ CFU/thigh. In vitro resistance to imipenem was confirmed in the in vivo study of these seven strains; and in the monotherapy group with the human-simulated imipenem dosing regimen, the mean increase in bacterial load was 2.16 ± 0.74 log₁₀ CFU/thigh. With the human-simulated dosing regime with the imipenem/Compound 1 of 500/250 mg (2:1), the isolated strains of *Acinetobacter* baumannii were all killed at 1-log, except for the strain ACB 160. In addition, five of the seven isolated strains were skilled at approximately 2-log.

### (3) Klebsiella pneumoniae

At 0 h, the mean bacterial load in the thighs of mice was 5.92 ± 0.14 log₁₀ CFU/thigh. Four isolated strains tested were all sufficiently proliferated in the neutropenic thigh infection model; and in the control mice administrated with an untreated vehicle, the mean increase in bacterial load at 4 h was 3.15±1.00 log1.00 CFU/thigh. In vitro resistance to imipenem was confirmed in the in vivo study of these seven strains; and in the monotherapy group with the human-simulated imipenem dosing regimen, the mean increase in bacterial load was 2.83 ± 0.81 log₁₀ CFU/thigh. With the human-simulated dosing regime with the imipenem/Compound 1 of 500/250 mg (2:1), all the strains tested reached or exceeded a bacterial growth cessation status, but none was killed at a mean level of 1-log.

In summary, in the neutropenic thigh infection model, the co-administration of imipenem/Compound 1 (500/250 mg, q6h, infused for 1 h) shows significant in vivo efficacy against serine carbapenemase-producing *Pseudomonas aeruginosa, Acinetobacter baumannii,* and **Klebsiella pneumoniae,** which provides support for the consideration of such a co-administration and dose in clinical trials aimed at treating serious infections caused by these bacteria.

The study on co-administration dose ratio in the above examples shows that Compound 1 can restore the in vivo antibacterial activity of imipenem against the tested drug-resistant strains at a test dose ratio of imipenem:Compound 1 as 1:1 to 10:1. At sufficient doses of imipenem and Compound 1, the co-administration dose ratio of imipenem:Compound 1 of 1:1 to 4:1 is an appropriate ratio for the strains of *Acinetobacter baumannii, Klebsiella pneumoniae* and *Pseudomonas aeruginosa,* and more further, the co-administration dose ratio of imipenem:Compound 1 of 1:1 or 1:2 is more optimal, which can be used in subsequent further non-clinical and clinical evaluations.

### Example 6: Selection of Solubilizer Type

The solubilization effect of sodium sulfobutylether-β-cyclodextrin (SBECD) and hydroxypropyl-β-cyclodextrin (HP-β-CD) on Compound 1 was investigated by taking the solubility in 50 mM pH 4.5 acetate buffer medium and water as a reference. The results showed that SBECD and HP-β-CD had a solubilization effect on Compound 1, with a better solubilization effect observed in SBECD. The results are shown in the table below.

**Table Solubility of Compound 1 incubated for 24 h at 25°C**

| Composition | Concentration (mg/mL) |
|---|---|
| Water + Compound 1 (control) | 0.6 |
| 50 mM acetate buffer (pH 4.5) + Compound 1 (control) | 3.85 |
| Aqueous HP-β-CD with the mass concentration of 10% + Compound 1 | 5.00 |
| Aqueous HP-β-CD with the mass concentration of 20% + Compound 1 | 6.40 |
| Aqueous HP-β-CD with the mass concentration of 30% + Compound 1 | 7.72 |
| Aqueous SBECD with the mass concentration of 10% + Compound 1 | 5.96 |
| Aqueous SBECD with the mass concentration of 20% + Compound 1 | 8.46 |
| Water + Compound 1 (control) | 0.6 |
| Aqueous SBECD with the mass concentration of 30% + Compound 1 | 10.49 |

In the art, surfactants may also be used for solubilization. To further improve the solubility of Compound 1, a surfactant was added on the basis of SBECD to investigate the solubility. The results showed that the added surfactant failed to significantly improve the solubility of Compound 1. The results are shown in the table below.

**Table Solubility of Compound 1 incubated for 24 h at 25°C**

| Composition | Concentration (mg/mL) |
|---|---|
| Aqueous SBECD with the mass concentration of 10% + Compound 1 | 5.96 |
| Aqueous SBECD with the mass concentration of 10% and aqueous PEG 400 with the mass concentration of 5% + Compound 1 | 6.21 |
| Aqueous SBECD with the mass concentration of 10% and aqueous PEG 400 with the mass concentration of 20% + Compound 1 | 6.35 |
| Aqueous SBECD with the mass concentration of 10% and aqueous PEG 400 with the mass concentration of 40% + Compound 1 | 6.84 |
| Aqueous SBECD with the mass concentration of 10% and aqueous PVP K12 with the mass concentration of 0.5% + Compound 1 | 6.44 |
| Aqueous SBECD with the mass concentration of 10% and aqueous PVP K12 with the mass concentration of 1% + Compound 1 | 6.42 |
| Aqueous SBECD with the mass concentration of 10% and aqueous PVP K12 with the mass concentration of 2.5% + Compound 1 | 6.38 |
| Aqueous SBECD with the mass concentration of 10% and aqueous Poloxamer 188 with the mass concentration of 1% + Compound 1 | 6.75 |
| Aqueous SBECD with the mass concentration of 10% and aqueous Solutol HS15 with the mass concentration of 1% + Compound 1 | 6.68 |
| Aqueous SBECD with the mass concentration of 10% and aqueous Tween 80 with the mass concentration of 1% + Compound 1 | 6.92 |
| Aqueous SBECD with the mass concentration of 10%, aqueous PVP K12 with the mass concentration of 0.5% and aqueous Poloxamer 188 with the mass concentration of 1% + Compound 1 | 6.95 |
| Aqueous SBECD with the mass concentration of 10%, aqueous PVP K12 with the mass concentration of 0.5% and aqueous Solutol HS15 with the mass concentration of 1% + Compound 1 | 6.87 |
| Aqueous SBECD with the mass concentration of 10%, aqueous PVP K12 with the mass concentration of 0.5% and aqueous Tween 80 with the mass concentration of 1% + Compound 1 | 6.99 |

### Example 7 Selection of Solubilizer Dosage

To develop a formulation of imipenem/cilastatin/Compound 1 for injection, the key is to increase the solubility of the raw material Compound 1 and improve the stability of Compound 1. At the dissolution temperature of 85°C to 90°C, the amount of SBECD that meets the dissolution amount of Compound 1 and the quality standard for the lyophilized powder of Compound 1 was investigated.

Recipes were designed with the mass ratios of Compound 1:SBECD as 1:5, 1:7.5, 1:10, and 1:12.5, respectively, with a solution preparation process of dissolving SBECD and Compound 1 in water for 10 minutes at 85°C to 90°C. The dissolution conditions in the solution preparation stage and their effects on the lyophilized powder of Compound 1 were investigated respectively. The results can be found in the table below.

**Table Effects of different dosages of SBECD on the solution and lyophilized powder of Compound 1**

| **Compou nd1: SBECD** | **Dissolution temperature** | **Solution of Compound 1** | | **Lyophilized powder of Compound 1** | | | |
|---|---|---|---|---|---|---|---|
| | | **Concentr ation of Compou nd 1 (mg/mL)** | **Dissoluti on condition** | **Reconsti tution of lyophiliz ed finished product** | **Wate r** | **Content of Compoun d 1 in 1 g of lyophilized powder of Compound 1** | **Total impu rities (%)** |
| 1:5 | 85°C~90°C | 4.00 | The raw material was partially precipitated and not completely dissolved | Failed in complete reconstitut ion, with relatively turbid solution | 3.2 % | 0.07838/8 | 0.94 |
| 1:7.5 | | 9.61 | Substantia lly completel y dissolved | Failed in complete reconstitut ion, with slight turbidity | 2.6% | 0.0914g/g | 0.84 |
| 1:10 | | 9.75 | Completel y dissolved | Successful reconstitut ion, with a clear solution | 2.2% | 0.0926g/g | 0.81 |
| 1:12.5 | | 9.90 | Completel y dissolved | Successful reconstitut ion, with a clear solution | 2.3% | 0.0932g/g | 0.79 |

When the ratio of Compound 1:SBECD is 1:5, the Compound 1 was failed in complete dissolution in the solution preparation stage, and the reconstitution solution of lyophilized powder resulting from lyophilization was turbid; and when the ratio of Compound 1:SBECD was 1:7.5, the Compound 1 was substantially completely dissolved in the solution preparation stage, and the reconstitution solution of lyophilized powder resulting from lyophilization was slightly turbid, which is also unacceptable. When the ratio of Compound 1:SBECD was 1:10 and 1:12.5, the Compound 1 could be completely dissolved, and the reconstitution solution of lyophilized powder resulting from lyophilization was also clear.

### Example 8

Preparation of Composition of Compound 1 as Injection (125 mg of specified Compound 1, 250 mg of imipenem, and 250 mg of cilastatin) The amounts of imipenem monohydrate and cilastatin sodium needed to be converted according to the molecular formula before feeding.

### Recipe (per 100 vials)

| | |
|---|---|
| **Compound 1** | **12.5 g** |
| **Sodium sulfobutylether-o-cyclodex⁻trin** | **35.0 g** |
| **Mannitol** | **70.0 g** |
| **Imipenem monohydrate** | **26.5 g** |
| **Cilastatin sodium** | **26.5 g** |
| **Sodium bicarbonate** | **1.0 g** |
| **Purified Water *¹** | **Add up to 3.5 L** |

| | |
|---|---|
| Note*1: The purified water was removed during lyophilization. | |

Preparation process: Add an appropriate amount of water to a beaker; add 35.0 g of sodium sulfobutylether-β-cyclodextrin and 70.0 g of mannitol to the beaker and stir them for dissolution; heat the mixture to 70°C in a water bath; add the raw material Compound 1 and stir the mixture for dissolution until clear; cool to room temperature, and then add 26.5 g of imipenem monohydrate, 26.5 g of cilastatin sodium, and 1.0 g of sodium bicarbonate until clear; filter the mixture with a 0.45 µm microporous filter membrane; and conduct split charging before lyophilization. The composition of Compound 1 was then obtained as an injection.

### Example 9

### Preparation of Composition of Compound 1 as Injection (50 mg of specified Compound 1, 100 mg of imipenem, and 100 mg of cilastatin)

Recipe (per 100 bottles, where the amounts of imipenem monohydrate and cilastatin sodium needed to be converted according to the molecular formula before feeding.)

| | |
|---|---|
| **Compound 1** | **5 g** |
| **Sodium sulfobutylether-β-cyclodextri** | **50 g** |
| **Anhydrous citric acid** | **0.77 g** |
| **Sodium hydroxide** | **0.25 g** |
| **Imipenem monohydrate** | **10.62 g** |
| **Cilastatin sodium** | **10.62 g** |
| **Sodium bicarbonate** | **0.4 g** |
| **Purified Water *¹** | **Add up to 1 L** |

| | |
|---|---|
| Note*1: The purified water was removed during lyophilization. | |

Preparation process: Add an appropriate amount of water to a beaker; add 50.0 g of sodium sulfobutylether-β-cyclodextrin, 0.77 g of anhydrous citric acid, and 0.25 g of sodium hydroxide to the beaker and stir the mixture for dissolution; heat the mixture to 70°C in a water bath; add the raw material Compound 1 and stir the mixture for dissolution until clear; cool to room temperature, and then filter the mixture with a 0.45 µm microporous filter membrane and then lyophilize the mixture; and crush, sieve and mix the lyophilized product. Add 10.62 g of imipenem monohydrate, 10.62 g of cilastatin sodium, and 0.4 g of sodium bicarbonate; mix them; and conduct split charging on the product. The composition of Compound 1 was then obtained as an injection.

### Example 10

Preparation of Composition of Compound 1 as Injection (250 mg of specified Compound 1, 500 mg of imipenem, and 500 mg of cilastatin, where the amounts of imipenem monohydrate and cilastatin sodium needed to be converted according to the molecular formula before feeding) Recipe (per 100 vials)

| | |
|---|---|
| **Compound 1** | **25.0 g** |
| **Sodium sulfobutylether-β-cyclodextrin** | **250.0 g** |
| **Anhydrous citric acid** | **3.85 g** |
| **Sodium hydroxide** | **1.25 g** |
| **Imipenem monohydrate** | **53.1 g** |
| **Cilastatin sodium** | **53.1 g** |
| **Sodium bicarbonate** | **2.0 g** |
| **Purified Water *¹** | **Add up to 2.5 L** |

| | |
|---|---|
| Note*1: The purified water was removed during lyophilization. | |

Preparation process: Add an appropriate amount of water to a beaker; add 250.0 g of sodium sulfobutylether-β-cyclodextrin, 3.85 g of anhydrous citric acid, and 1.25 g of sodium hydroxide to the beaker and stir the mixture for dissolution; heat the mixture to 80°C in a water bath; add the raw material Compound 1 and stir the mixture for dissolution until clear; cool to room temperature, and then filter the mixture with a 0.45 µm microporous filter membrane and then lyophilize the mixture; and crush, sieve and mix the lyophilized product. Add 53.1 g of imipenem monohydrate, 53.1 g of cilastatin sodium, and 2.0 g of sodium bicarbonate; mix them; and condcut split charging on the product. The composition of Compound 1 was then obtained as an injection. The following provides the solubility data of the composition as an injection at different temperatures:

| Dissolution temperature (°C) | Dissolution time (min) | Concentration of Compound 1 (mg/mL) | Total impurities (%) |
|---|---|---|---|
| **70** | **5** | **7.31** | **0.69** |
| | **10** | **8.11** | **0.74** |
| | **20** | **8.43** | **0.80** |
| | **30** | **8.48** | **0.93** |
| **75** | **5** | **8.20** | **0.72** |
| | **10** | **8.82** | **0.85** |
| | **20** | **9.17** | **0.96** |
| | **30** | **9.25** | **1.1** |
| **80** | **5** | **8.88** | **0.70** |
| | **10** | **9.50** | **0.89** |
| | **20** | **9.75** | **0.96** |
| | **30** | **9.79** | **1.1** |
| **85** | **5** | **9.74** | **0.71** |
| | **10** | **10.08** | **0.83** |
| | **20** | **10.07** | **1.1** |
| | **30** | **10.08** | **1.4** |
| **90** | **5** | **9.87** | **0.82** |
| | **10** | **10.03** | **0.96** |
| | **20** | **9.94** | **1.3** |
| | **30** | **9.95** | **1.7** |

### Example 11

Preparation of Composition of Compound 1 as Injection (250 mg of specified Compound 1, 500 mg of imipenem, and 500 mg of cilastatin, where the amounts of imipenem monohydrate and cilastatin sodium needed to be converted according to the molecular formula before feeding) Recipe (per 100 vials)

| | |
|---|---|
| **Compound 1** | **25.0 g** |
| **Sodium sulfobutylether-β-cyclodextrin** | **225.0 g** |
| **Imipenem monohydrate** | **53.1 g** |
| **Cilastatin sodium** | **53.1 g** |
| **Sodium bicarbonate** | **2.5 g** |
| **Purified Water *¹** | **Add up to 2.5 L** |

| | |
|---|---|
| Note*1: The purified water was removed during lyophilization. | |

Preparation process: Add an appropriate amount of water to a beaker; add 225.0 g of sodium sulfobutylether-β-cyclodextrin to the beaker and stir the mixture for dissolution; heat the mixture to 85°C in a water bath; add the raw material Compound 1 and stir the mixture for dissolution until clear; cool to room temperature, and then filter the mixture with a 0.45 µm microporous filter membrane and then lyophilize the mixture; and crush, sieve and mix the lyophilized product. Add 53.1 g of imipenem monohydrate, 53.1 g of cilastatin sodium, and 2.5 g of sodium bicarbonate; mix them; and conduct split charging on the product. The composition of Compound 1 was then obtained as an injection.

### Example 12

Preparation of Composition of Compound 1 as Injection (150 mg of specified Compound 1, 300 mg of imipenem, and 300 mg of cilastatin sodium, where the amounts of imipenem monohydrate and cilastatin sodium needed to be converted according to the molecular formula before feeding)

### Recipe (per 100 vials)

| | |
|---|---|
| **Compound 1** | **15.0 g** |
| **Sodium sulfobutylether-β-cyclodextrin** | **120.0 g** |
| **Imipenem monohydrate** | **15.93 g** |
| **Cilastatin sodium** | **15.93 g** |
| **Sodium bicarbonate** | **1.2 g** |
| **Purified Water *¹** | **Add up to 1.5 L** |

| | |
|---|---|
| Note*1: The purified water was removed during lyophilization. | |

Preparation process: Add an appropriate amount of water to a beaker; add 120.0 g of sodium sulfobutylether-β-cyclodextrin to the beaker and stir the mixture for dissolution; heat the mixture to 85°C in a water bath; add the raw material Compound 1 and stir the mixture for dissolution until clear; cool to room temperature, and then filter the mixture with a 0.45 µm microporous filter membrane and then lyophilize the mixture; and crush, sieve and mix the lyophilized product. Add 15.93 g of imipenem monohydrate, 15.93 g of cilastatin sodium, and 1.2 g of sodium bicarbonate; mix them; and conduct split charging on the product. The composition of Compound 1 was then obtained as an injection.

### Example 13

Preparation of Composition of Compound 1 as Injection (250 mg of specified Compound 1, 500 mg of imipenem, and 500 mg of cilastatin, where the amounts of imipenem monohydrate and cilastatin sodium needed to be converted according to the molecular formula before feeding) Recipe (per 100 vials)

| | |
|---|---|
| **Compound 1** | **25.0 g** |
| **Sodium sulfobutylether-β-cyclodextrin** | **250.0 g** |
| **Polyvidone** | **1.25 g** |
| **Imipenem monohydrate** | **53.1 g** |
| **Cilastatin sodium** | **53.1 g** |
| **Sodium bicarbonate** | **2.0 g** |
| **Purified Water *¹** | **Add up to 2.5 L** |

| | |
|---|---|
| Note*1: The purified water was removed during lyophilization. | |

Preparation process: Add an appropriate amount of water to a beaker; add 250.0 g of sodium sulfobutylether-β-cyclodextrin to the beaker and stir the mixture for dissolution; heat the mixture to 85°C in a water bath; add the raw material Compound 1 and stir the mixture for dissolution until clear; cool to room temperature, and then add 1.25 g of polyvidone; filter the mixture with a 0.45 µm microporous filter membrane and then lyophilize the mixture; and crush, sieve and mix the lyophilized product. Add 53.1 g of imipenem monohydrate, 53.1 g of cilastatin sodium, and 2.0 g of sodium bicarbonate; mix them; and conduct split charging on the product. The composition of Compound 1 was then obtained as an injection.

### Example 14

Preparation of Composition of Compound 1 as Injection (250 mg of specified Compound 1, 500 mg of imipenem, and 500 mg of cilastatin, where the amounts of imipenem monohydrate and cilastatin sodium needed to be converted according to the molecular formula before feeding) Recipe (per 100 vials)

| | |
|---|---|
| **Compound 1** | **25.0 g** |
| **Sodium sulfobutylether-β-cyclodextrin** | **250.0 g** |
| **Polyvidone** | **5.0 g** |
| **Imipenem monohydrate** | **53.1 g** |
| **Cilastatin sodium** | **53.1 g** |
| **Sodium bicarbonate** | **2.0 g** |
| **Purified Water *¹** | **Add up to 2.5 L** |

| | |
|---|---|
| Note*1: The purified water was removed during lyophilization. | |

Preparation process: Add an appropriate amount of water to a beaker; add 250.0 g of sodium sulfobutylether-β-cyclodextrin to the beaker and stir the mixture for dissolution; heat the mixture to 85°C in a water bath; add the raw material Compound 1 and stir the mixture for dissolution until clear; cool to room temperature, and then add 5.0 g of polyvidone; filter the mixture with a 0.45 µm microporous filter membrane and then lyophilize the mixture; and crush, sieve and mix the lyophilized product. Add 53.1 g of imipenem monohydrate, 53.1 g of cilastatin sodium, and 2.0 g of sodium bicarbonate; mix them; and conduct split charging on the product. The composition of Compound 1 was then obtained as an injection.

From the perspective of drug safety, the amount of solubilizer used in the injection should be lower than the maximum single-dose amount as specified in the FDA IIG database as much as possible. That is, in the FDA IIG database, the maximum single-dose amount of sodium sulfobutylether-β-cyclodextrin is 3200 mg, and the maximum allowed daily dose is 15 g. Accordingly, the ratio of imipenem/cilastatin/Compound 1 is determined to be 2:2:1, and preferably, each bottle contains imipenem/cilastatin/compound at 1250 mg/250 mg/125 mg and 500 mg/500 mg/250 mg.

## Claims

1. A pharmaceutical composition A, wherein active ingredients of the pharmaceutical composition A comprise a substance A and a substance B;
the substance A is a compound 1 or a solvate thereof;
the substance B is imipenem or a solvate thereof;
a mass ratio of the substance B to the substance A is 1:1 to 10:1; the substance B has a mass calculated on the basis of the imipenem; the substance A has a mass calculated on the basis of the compound 1; and
the compound 1 has a structural formula shown below:

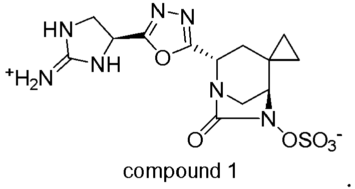

2. The pharmaceutical composition A according to claim 1, wherein the pharmaceutical composition A satisfies one or more of the following conditions:
(1) the active ingredients of the pharmaceutical composition A consist of the substance A and the substance B;
(2) in the pharmaceutical composition A, the substance B is imipenem monohydrate; and
(3) the mass ratio of the substance B to the substance A is 1:1, 2:1, 3:1, 4:1, 5:1, 7:1 or 10:1.

3. The pharmaceutical composition A according to claim 1, wherein the mass ratio of the substance B to the substance A is 1:1 to 4:1, preferably 1:1 to 2:1, more preferably 2:1.

4. The pharmaceutical composition A according to claim 1, wherein the pharmaceutical composition A satisfies one or more of the following conditions:
(1) in the pharmaceutical composition A, the substance A is the compound 1;
(2) in the pharmaceutical composition A, the compound 1 has a mass percentage of 2% to 15%, preferably 5% to 10%, for example 6.44%, 6.97%, 7.29%, 6.50% or 8.93%; and
(3) in the pharmaceutical composition A, the substance B is imipenem monohydrate, and the imipenem monohydrate has a mass percentage of preferably 9% to 16%, for example, 13.81%, 13.68%, 15.45%, 13.67%, 14.80% or 9.48%.

5. The pharmaceutical composition A according to claim 1, wherein the pharmaceutical composition A satisfies one or more of the following conditions:
(1) in the pharmaceutical composition A, the imipenem has a mass percentage of 5% to 20%, preferably 8% to 15%; and
(2) the pharmaceutical composition A further comprises a pharmaceutically acceptable excipient.

6. The pharmaceutical composition A according to claim 5, wherein in the pharmaceutical composition A, the pharmaceutically acceptable excipient comprises a solubilizer, and can further comprise one or two of a filler and a pH regulator.

7. The pharmaceutical composition A according to claim 6, wherein the pharmaceutical composition A satisfies one or more of the following conditions:
(1) in the pharmaceutical composition A, the solubilizer is selected from one or more of sodium sulphobutylether-β-cyclodextrin, sodium hydroxypropyl-β-cyclodextrin, meglumine and polysorbate 80, preferably sodium sulphobutylether-β-cyclodextrin and/or sodium hydroxypropyl-β-cyclodextrin , more preferably sodium sulphobutylether-β-cyclodextrin;
(2) in the pharmaceutical composition A, the solubilizer has a mass percentage of 20% to 70%, for example, 20.41%, 64.38%, 62.73%, 65.03%, 64.40% or 71.40%, preferably 50% to 70%;
(3) in the pharmaceutical composition A, a mass ratio of the substance A to the solubilizer is 1:(2 to 13), for example 1:2.8, 1:5, 1:7.5, 1:8, 1:9, 1:10 or 1:12.5, preferably 1:(10 to 13), and the substance A has a mass calculated on the basis of the compound 1;
(4) when the pharmaceutically acceptable excipient further comprises a filler, in the pharmaceutical composition A, the filler is selected from one or more of mannitol, lactose, sucrose, microcrystalline cellulose, glucose, fructose, povidone and trehalose, preferably mannitol and/or povidone;
(5) when the pharmaceutically acceptable excipient further comprises a filler, in the pharmaceutical composition A, the filler has a mass percentage of 0.3% to 70%, for example, 30% to 70%, preferably 35% to 50%, for another example, 0.33%, 1.29% or 40.82%;
(6) when the pharmaceutically acceptable excipient further comprises a pH regulator, in the pharmaceutical composition A, the pH regulator is selected from one or more of anhydrous citric acid, sodium hydroxide, sodium bicarbonate, anhydrous disodium hydrogen phosphate, sodium dihydrogen phosphate, disodium tartrate, maleic acid, magnesium hydroxide and calcium hydroxide, and preferably selected from one or more of sodium bicarbonate, sodium hydroxide and anhydrous citric acid;
(7) when the pharmaceutically acceptable excipient further comprises a pH regulator, in the pharmaceutical composition A, the pH regulator has a mass percentage of 0% to 2.0%, for example, 0.52%, 0.58%, 0.70%, 0.71% or 1.83%, preferably 0.3% to 0.7%; and
(8) the pharmaceutical composition A exists as an injection, for example, lyophilized powder.

8. Use of the pharmaceutical composition A according to any one of claims 1 to 7 in preparation of a medicament for inhibiting bacteria, wherein the bacteria are resistant to β-lactam antibiotics; and the bacteria resistant to β-lactam antibiotics are preferably selected from one or more of *Pseudomonas aeruginosa*, *Acinetobacter* baumannii and *Klebsiella pneumoniae.*

9. Use of a substance A in preparation of a medicament for inhibiting bacteria, wherein the substance A is used in combination with a substance B; the substance B and the substance A have definitions and dosages as defined in any one of claims 1 to 5; and the bacteria are as defined in claim 8.

10. Use of a substance B in preparation of a medicament for inhibiting bacteria, wherein the substance B is used in combination with a substance A; the substance B and the substance A have definitions and dosages as defined in any one of claims 1 to 5; and the bacteria are as defined in claim 8.

11. A pharmaceutical composition B, wherein active ingredients of the pharmaceutical composition B comprise a substance A, a substance B and a substance C;
the substance A is a compound 1 or a solvate thereof;
the substance B is imipenem or a solvate thereof;
the substance C is cilastatin sodium or a solvate thereof;
a mass ratio of the substance C to the substance B to the substance A is (1 to 10):(1 to10):1; the substance C has a mass calculated on the basis of cilastatin; the substance B has a mass calculated on the basis of the imipenem; the substance A has a mass calculated on the basis of the compound 1; and
the compound 1 has a structural formula shown below:

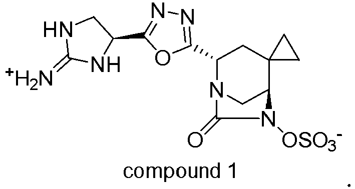

12. The pharmaceutical composition B according to claim 11, wherein the pharmaceutical composition B satisfies one or more of the following conditions:
(1) the active ingredients of the pharmaceutical composition B consist of the substance A, the substance B and the substance C;
(2) in the pharmaceutical composition B, the substance B is imipenem monohydrate;
(3) in the pharmaceutical composition B, the substance C is the cilastatin sodium; and
(4) the mass ratio of the substance C to the substance B to the substance A is 1:1:1, 2:2:1, 3:3:1, 4:4:1, 5:5:1, 7:7:1 or 10:10:1.

13. The pharmaceutical composition B according to claim 11, wherein the mass ratio of the substance C to the substance B to the substance A is (1 to 4):(1 to 4):1, preferably (1 to 2):(1 to 2):1, more preferably 2:2:1.

14. The pharmaceutical composition B according to claim 11, wherein the pharmaceutical composition B satisfies one or more of the following conditions:
(1) in the pharmaceutical composition B, the substance A is the compound 1;
(2) in the pharmaceutical composition B, the compound 1 has a mass percentage of 2% to 15%, preferably 5% to 10%, for example 6.44%, 6.50%, 6.97%, 7.29% or 8.93%;
(3) in the pharmaceutical composition B, the substance B is imipenem monohydrate, and the imipenem monohydrate has a mass percentage of preferably 9% to 16%, for example, 13.81%, 13.68%, 15.45%, 13.67%, 14.80% or 9.48%; and
(4) in the pharmaceutical composition B, the substance C exists as amorphous cilastatin sodium, and the cilastatin sodium has a mass percentage of preferably 9% to 16%, for example, 13.81%, 13.68%, 9.48%, 13.67%, 14.80% or 15.45%.

15. The pharmaceutical composition B according to claim 11, wherein the pharmaceutical composition B satisfies one or more of the following conditions:
(1) in the pharmaceutical composition B, the imipenem has a mass percentage of 5% to 20%, preferably 8% to 15%;
(2) in the pharmaceutical composition B, the cilastatin sodium has a mass percentage of 5% to 20%, preferably 8% to 15%, and the cilastatin sodium has a mass calculated on the basis of cilastatin; and
(3) the pharmaceutical composition B further comprises a pharmaceutically acceptable excipient.

16. The pharmaceutical composition B according to claim 15, wherein in the pharmaceutical composition B, the pharmaceutically acceptable excipient comprises a solubilizer, and can further comprise one or two of a filler and a pH regulator.

17. The pharmaceutical composition B according to claim 16, wherein the pharmaceutical composition B satisfies one or more of the following conditions:
(1) in the pharmaceutical composition B, the solubilizer is selected from one or more of sodium sulphobutylether-β-cyclodextrin, sodium hydroxypropyl-β-cyclodextrin, meglumine and polysorbate 80, preferably sodium sulphobutylether-β-cyclodextrin and sodium hydroxypropyl-β-cyclodextrin , more preferably sodium sulphobutylether-β-cyclodextrin;
(2) in the pharmaceutical composition B, the solubilizer has a mass percentage of 20% to 70%, for example, 20.41%, 64.38%, 62.73%, 65.03%, 64.40% or 71.40%, preferably 50% to 70%;
(3) in the pharmaceutical composition B, a mass ratio of the substance A to the solubilizer is 1:(2 to 13), for example 1:2.8, 1:5, 1:7.5, 1:8, 1:9, 1:10 or 1:12.5, preferably 1:(10 to 13), and the substance A has a mass calculated on the basis of the compound 1;
(4) when the pharmaceutically acceptable excipient further comprises a filler, in the pharmaceutical composition B, the filler is selected from one or more of mannitol, lactose, sucrose, microcrystalline cellulose, glucose, fructose, povidone and trehalose, preferably mannitol and/or povidone;
(5) when the pharmaceutically acceptable excipient further comprises a filler, in the pharmaceutical composition B, the filler has a mass percentage of 0.3% to 70%, for example, 30% to 70%, preferably 35% to 50%, for another example, 0.33%, 1.29% or 40.82%;
(6) when the pharmaceutically acceptable excipient further comprises a pH regulator, in the pharmaceutical composition B, the pH regulator is selected from one or more of anhydrous citric acid, sodium hydroxide, sodium bicarbonate, anhydrous disodium hydrogen phosphate, sodium dihydrogen phosphate, disodium tartrate, maleic acid, magnesium hydroxide and calcium hydroxide, and preferably selected from one or more of sodium bicarbonate, anhydrous citric acid and sodium hydroxide; and
(7) when the pharmaceutically acceptable excipient further comprises a pH regulator, in the pharmaceutical composition B, the pH regulator has a mass percentage of 0% to 2.0%, for example, 0.52%, 0.58%, 0.70%, 0.71% or 1.83%, preferably 0.3% to 0.7%.

18. The pharmaceutical composition B according to claim 11, wherein the pharmaceutical composition B satisfies any of the following conditions:
(1) the pharmaceutical composition B comprises the compound 1, the sodium sulphobutylether-β-cyclodextrin, the mannitol, the imipenem monohydrate, the cilastatin sodium and the sodium bicarbonate;
(2) the pharmaceutical composition B comprises the compound 1, the sodium sulphobutylether-β-cyclodextrin, the anhydrous citric acid, the sodium hydroxide, the imipenem monohydrate, the cilastatin sodium and the sodium bicarbonate;
(3) the pharmaceutical composition B comprises the compound 1, the sodium sulphobutylether-β-cyclodextrin, the imipenem monohydrate, the cilastatin sodium and the sodium bicarbonate; or
(4) the pharmaceutical composition B comprises the compound 1, the sodium sulphobutylether-β-cyclodextrin, the povidone, the imipenem monohydrate, the cilastatin sodium and the sodium bicarbonate.

19. The pharmaceutical composition B according to claim 11, wherein the pharmaceutical composition B satisfies any of the following conditions:
(1) the pharmaceutical composition B comprises 7.29% of the compound 1, 20.41% of the sodium sulphobutylether-β-cyclodextrin, 40.82% of the mannitol, 15.45% of the imipenem monohydrate, 15.45% of the cilastatin sodium and 0.58% of the sodium bicarbonate, in percentage by mass;
(2) the pharmaceutical composition B comprises 6.44% of the compound 1, 64.38% of the sodium sulphobutylether-β-cyclodextrin, 0.99% of the anhydrous citric acid, 0.32% of the sodium hydroxide, 13.67% of the imipenem monohydrate, 13.67% of the cilastatin sodium and 0.52% of the sodium bicarbonate, in percentage by mass;
(3) the pharmaceutical composition B comprises 6.97% of the compound 1, 62.73% of the sodium sulphobutylether-β-cyclodextrin, 14.80% of the imipenem monohydrate, 14.80% of the cilastatin sodium and 0.70% of the sodium bicarbonate, in percentage by mass;
(4) the pharmaceutical composition B comprises 8.93% of the compound 1, 71.40% of the sodium sulphobutylether-β-cyclodextrin, 9.48% of the imipenem monohydrate, 9.48% of the cilastatin sodium and 0.71% of the sodium bicarbonate, in percentage by mass;
(5) the pharmaceutical composition B comprises 6.50% of the Compound 1, 65.03% of the sodium sulphobutylether-β-cyclodextrin, 0.33% of the povidone, 0.52% of the sodium bicarbonate, 13.81% of the imipenem monohydrate, and 13.81% of the cilastatin sodium, in percentage by mass; or
(6) the pharmaceutical composition B comprises 6.44% of the compound 1, 64.40% of the sodium sulphobutylether-β-cyclodextrin, 1.29% of the povidone, 0.52% of the sodium bicarbonate, 13.68% of the imipenem monohydrate, and 13.68% of the cilastatin sodium, in percentage by mass.

20. The pharmaceutical composition B according to claim 11, wherein the pharmaceutical composition B satisfies one or more of the following conditions:
(1) the pharmaceutical composition B exists as an injection; and
(2) when the pharmaceutical composition B exists as an injection, the imipenem/cilastatin/compound 1 in the pharmaceutical composition B has a content of 250 mg/250 mg/125 mg or 500 mg/500 mg/250 mg.

21. Use of the pharmaceutical composition B according to claim 11 in preparation of a medicament for inhibiting bacteria, wherein the bacteria are resistant to β-lactam antibiotics; and the bacteria resistant to β-lactam antibiotics are preferably selected from one or more of *Pseudomonas aeruginosa*, *Acinetobacter* baumannii and *Klebsiella pneumoniae.*

22. Use of a substance A in preparation of a medicament for inhibiting bacteria, wherein the substance A is used in combination with a substance B and a substance C; the substance C, the substance B and the substance A have definitions and dosages as defined in any one of claims 11 to 15; and the bacteria are as defined in claim 21.

23. Use of a substance B in preparation of a medicament for inhibiting bacteria, wherein the substance B is used in combination with a substance A and a substance C; the substance C, the substance B and the substance A have definitions and dosages as defined in any one of claims 11 to 15; and the bacteria are as defined in claim 21.

24. Use of a substance C in preparation of a medicament for inhibiting bacteria, wherein the substance C is used in combination with a substance A and a substance B; the substance C, the substance B and the substance A have definitions and dosages as defined in any one of claims 11 to 15; and the bacteria are as defined in claim 21.
